# EUROPEAN PATENT APPLICATION

(11) **EP 4 563 070 A1**
(43) Date of publication of application: **04.06.2025**
(21) Application number: 24216617.1
(22) Date of filing: 29.11.2024
(51) Int. Cl.: A61B 3/04

(54) **MEASURING INSTRUMENT FOR DETERMINING THE NECESSARY CORRECTION OF A LENS TO IMPROVE VISION**

(30) Priority: 30.11.2023 NL 2036386; 27.04.2024 NL 2037567
(71) Applicant: Frambach B.V., 2332 AV Leiden (NL)
(72) Inventor: FRAMBACH, Christiaan Jacobus Hubertus, 2719SK Zoetermeer (NL)
(74) Representative: Verhees, Godefridus Josephus Maria

(57) **Abstract**

The measuring instrument has a measuring lens that has two different wavefronts in at least two directions that are perpendicular to each other with strengths that gradually increase from a value of -W to a value of +W. The measuring instrument also has a handle that is held up in two different positions that differ by 45 degrees during the determination of the amount of higher order aberrations to be corrected. With the handle in the direction of the wavefront, the strength of the wavefront is determined by turning the handle 180 degrees around the axis. By turning the position of the handle 45 degrees, the axis direction of the wavefront is determined by turning the handle 180 degrees around the axis. This measuring instrument can be used to determine the corrective value of a spectacle lens and contact lens, which can be used to correct higher order aberrations and is also useful in refractive surgery.

## Description

### Technical field of the invention

The invention relates to a measuring instrument for determining the necessary correction of a lens (spectacle lens, contact lens or eye lens) to reduce the negative effect on vision due to aberrations, comprising a measuring lens having a first strength in a first direction in the plane of the measuring lens and through the centre of the measuring lens and having a second strength in a second direction at an angle to the first direction in the plane of the measuring lens and through the centre of the measuring lens, which measuring instrument further comprises a straight handle connected to the measuring lens and extending in the plane of the measuring lens, the centre line of the handle passing through the centre of the measuring lens and extending along a line dividing an angle between the two said directions into two equal parts.

### Background of the invention

Such a measuring instrument is generally known under the name Jackson cross cylinder. The Jackson cross cylinder has a measuring lens whose surface is formed by two perpendicular cylinder surfaces, a cylinder with a positive power and a cylinder with a negative power (concave and convex shape). The absolute values of the powers of the cylinders are equal to each other. The Jackson cross cylinder also has a straight handle that is connected to the measuring lens and that extends in a direction at an angle of 45 degrees between the two axes of the cylinders. The measuring lens of the Jackson cross cylinder is provided with points or lines to indicate the axis of minus and plus powers.

The Jackson cross cylinder is used in subjective refraction to determine the power of the corrective lens and its axis in patients with astigmatism. In the eye test after spherical refraction, the measuring lens of the Jackson cross cylinder is first held in front of the cylinder and then rotated 180 degrees around the axis of the handle. In this way, the power of the cylinder is determined. Then the position of the handle is rotated 45 degrees. By rotating the glass 180 degrees around the axis of the handle again, the axis direction of the cylinder is determined.

With this Jackson cross cylinder it is possible to determine the lower order aberrations of the eye with the help of subjective refraction. In optics, cylindrical lenses are prescribed in addition to spherical lenses. The cylindrical lens has a constant strength in one direction and no strength in the direction perpendicular to it, or a plano strength. A cylindrical lens can be combined with a spherical lens. The spherical strength can be performed in plus or minus. Lower order aberrations can therefore be easily corrected with spherical lenses with the currently available lenses and can be combined with cylindrical lenses. In the known measuring instrument, the strengths are cylindrical strengths whose absolute values are equal but which differ in sign (positive/negative), whereby the axes of the cylinders are perpendicular to each other. In this case, both cylindrical shapes can be present in the surface on one side of the measuring lens or one of the cylindrical shapes can be present in each of the surfaces on both sides of the measuring lens.

Spectacle lenses are also already available that can correct higher order aberrations. Higher order aberrations can be determined using aberrometry. The current state of the art uses the values measured with aberrometry to supplement them as an added value to the spectacle correction. This measuring method does not use the experience of the patient being measured and is called an objective method.

From US2008/0143960A1 a diagnostic device is known that contains a set of lenses as described by Zernike. This patent application does not disclose how this set of lenses can be used to arrive at a prescription. The handle attached to the frame is only used to hold the frame while inserting a lens into the frame.

### Summary of the invention

An object of the invention is to provide a measuring instrument with which, on the basis of subjective refraction, the required amount of higher order aberration correction of a lens can be determined in order to improve vision. To this end, the measuring instrument according to the invention is characterized in that the measuring lens has a changing strength in at least one of the aforementioned directions. Preferably, the measuring lens has a gradually changing strength or shape without jumpy transitions.

The measuring lens of the known measuring instrument has a shape of the concave and convex side of a cylinder jacket in two directions at right angles to each other. This shape does not change in the aforementioned directions but is constant in these directions. By providing a test lens with a surface whose shape varies in the said directions and preferably varies gradually, the necessary corrections of a lens for higher order aberrations (coma, trefoil and secondary astigmatism) of the eye can also be determined. The result of a determination of the correction with the measuring instrument according to the invention can be used in the production of spectacle lenses and contact lenses and can also be used for refractive surgery.

The measuring lens of the measuring instrument according to the invention has a surface that is not shaped in the form of the concave or convex side of a cylinder jacket as in the measuring lens of the known measuring instrument, but has a shape that deviates from it.

The patient is asked to compare the effect of a measuring lens in different positions and to choose the best image. The method corresponds to the determination of the cylinder strength and the axis direction that we already know in subjective refraction. The advantage of this method is that the end result is achieved through the cooperation of the refractionist and the patient. During this subjective refraction, it is immediately taken into account that the patient processes the image in the visual cortex. This gives the patient influence on the desired spectacle correction. The final result can be assessed directly by the patient at the end of the measurement. The refractionist and patient must be well attuned to each other in order to achieve a good result. The outcome of the measurement with the measuring lens according to the invention can be combined with a spherocylindrical value that may have been determined earlier.

In order to arrive at a prescription, the measuring instrument according to the invention is provided with a handle with which the measuring lens can be turned around in front of the eye, just like with the Jackson cylinder. The center line of the handle of the diagnostic device known from US2008/0143960A1 does not extend along a line that bisects an angle between the two directions in which the strength varies.

An embodiment of the measuring instrument according to the invention is characterised in that the measuring lens has a strength that varies from a positive strength to a negative strength in at least two directions at an angle to each other. The maximum value of the positive strength and the absolute value of the minimum value of the negative strength can differ from each other (which is not the case with the diagnostic device known from US2008/0143960A1) or can be equal to each other.

Here too, the surface on one side of the measuring lens can be provided with a strength that runs in one direction and the surface on the opposite side can be provided with a strength that runs in the other direction or both directions can be present in the surface on one side (whereby the surface on the other side is flat).

An embodiment of this is characterized in that the first and second direction are perpendicular to each other. This measuring lens is suitable for determining the required correction of a spectacle lens in order to reduce the negative effect on vision due to coma in particular as a higher order aberration during subjective refraction.

A further embodiment of this is characterised in that the measuring lens also has a graduated strength in a third direction in the plane of the measuring lens, which is at an angle to the first and second direction, from a positive strength to a negative strength. Here too, the maximum value of the positive strength and the absolute value of the minimum value of the negative strength can differ from each other or be equal to each other, whereby the angles between the directions are equal, and whereby the progression of the strength in two neighbouring directions is opposite to each other. This measuring lens is suitable for determining the required correction of a spectacle lens in order to reduce the negative effect on vision due in particular to trefoil as a higher order aberration during subjective refraction. The measuring lenses described above with a gradually graduated strength in at least two directions have a shape of a wavefront. A wavefront in a spectacle lens is formed by changing it from a negative to a positive strength, whereby the centre of the spectacle lens has no strength. Optically, the correction proceeds from a horizontal centerline with a plano strength upwards with increasingly negative strength, while the same happens at the bottom, but then increasingly positive and that the increase in strength upwards proceeds at the same rate as the increase in strength downwards. Such a wavefront has already been described earlier by the Dutch physicist Frits Zernike.

A further embodiment of the measuring instrument according to the invention is characterized in that quadrants of the measuring lens have two different spherical strengths that are equal in absolute values but differ in sign, whereby the strength of neighboring quadrants differs in sign. This measuring lens is suitable for the determining the required correction of a spectacle lens to reduce the negative effect on vision due to secondary astigmatism in particular as a higher order aberration during subjective refraction.

The invention also relates to an assembly of a measuring instrument and a fitting set of fitting lenses. The measuring instrument of this assembly has a measuring lens that has a varying strength in two directions at right angles to each other from a positive strength to a negative strength, whereby the maximum value of the positive strength and the absolute value of the minimum value of the negative strength are equal to each other. Each fitting lens of this assembly has a varying strength in a first direction in the plane of the fitting lens and through the centre of the fitting lens from a positive strength to a negative strength, whereby the maximum value of the positive strength and the absolute value of the minimum value of the negative strength are equal to each other, and which fitting lens has no strength in a second direction at an angle to the first direction in the plane of the fitting lens and through the centre of the fitting lens.

### Brief description of the drawings

The invention will be further elucidated below on the basis of drawings. These drawings show an embodiment of the measuring instrument according to the present invention. In the drawings:
Figure 1 shows the known measuring instrument provided with a measuring lens with two different cylindrical strengths in directions perpendicular to each other, schematically represented;
Figure 2 shows the topography of the measuring lens of the known measuring instrument shown in figure 1;
Figure 3 shows a first embodiment of the measuring instrument according to the invention provided with a measuring lens with two wave fronts varying in strength in directions perpendicular to each other, schematically represented;
Figure 4 shows the topography of the measuring lens of the measuring instrument shown in figure 3;
Figure 5 shows a measuring lens according to the invention that can be used as a test lens for determining the required strength;
Figure 6 shows the topography of the measuring lens shown in figure 5;
Figure 7 shows a second embodiment of the measuring instrument according to the invention provided with a measuring lens with three wave fronts varying in strength, schematically represented;
Figure 8 shows the topography of the measuring lens of the measuring instrument shown in figure 7;
Figure 9 shows a third embodiment of the measuring instrument according to the invention provided with a measuring lens with different spherical strengths in the quadrants, schematically shown; and
Figure 10 shows the topography of the measuring lens of the measuring instrument shown in figure 9.

### Detailed description of the drawings

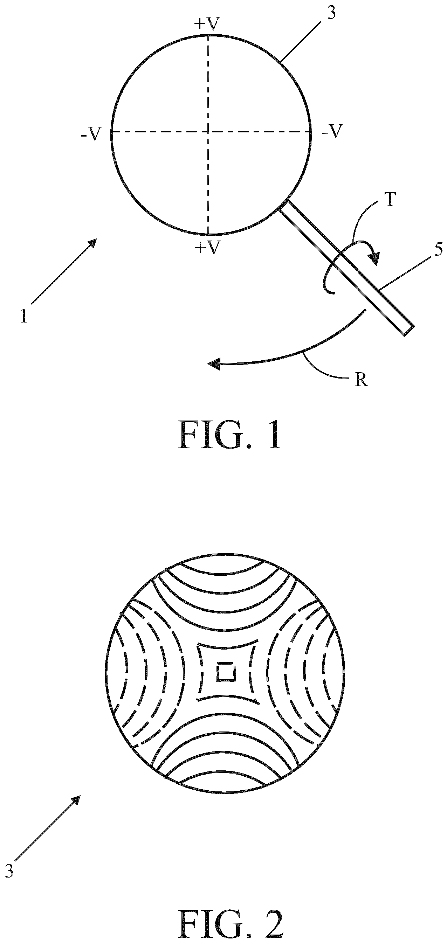

Figure 1 shows the known measuring instrument schematically. The measuring instrument 1 has a measuring lens 3 and a handle 5 that is connected to the measuring lens 3. The measuring lens 3 of this measuring instrument 1 has two different (cylindrical) strengths with equal values V but with different signs in two directions that lie at right angles to each other. The measuring lens 3 is best used after determining the spherical strength. The handle 5 is always held in two different positions that differ by 45 degrees during the determination of the amount of aberrations to be corrected. With the handle 5 in the direction as shown in figure 1, the strength of the cylinder is determined by turning the handle 5 180 degrees about an axis of the handle, arrow T. By turning the handle 5 about an axis perpendicular to and through the centre of the measuring lens 3 by 45 degrees, arrow R, the axis direction of the cylinder is determined by turning the handle 5 again 180 degrees about the axis of the handle 5. This known measuring instrument is used to correct astigmatism.

Figure 2 shows the topography of the measuring lens 3 of the known measuring instrument. The lines are contour lines of the measuring lens, with solid lines representing contour lines of a positive strength and broken lines representing contour lines of a negative strength. The measuring lens 3 is optically constructed as follows. The measuring lens 3 has two different cylindrical strengths in two different directions at right angles to each other, of which the strength in one of the directions has a value of +V and in the other direction the strength has a value of -V.

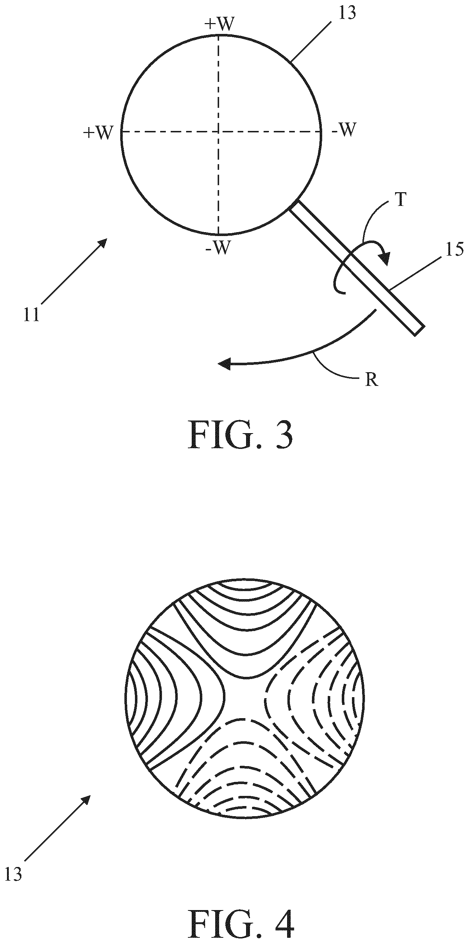

Figure 3 shows a first embodiment of the measuring instrument 11 according to the invention, schematically. The measuring lens 13 of this measuring instrument 11 has two different wavefronts in two directions at right angles to each other with strengths that gradually increase from a value of -W to a value of +W (a wavefront is when the strength varies in one direction). During the determination of the amount of higher order aberrations to be corrected, the handle 15 is also held up in two different positions that differ by 45 degrees from each other. With the handle in the direction of the wavefront (figure 3) the strength of the wavefront is determined by turning the handle around the axis 180, arrow T. By turning the position of the handle by 45 degrees, arrow R, the axis direction of the wavefront is determined by turning the handle again 180 degrees around the axis of the handle 15.

Figure 4 shows the topography of the measuring lens 13 of the first embodiment of the measuring instrument 11 according to the invention. A wavefront in the measuring lens 13 is optically constructed by giving the strength of the measuring lens a positive value +W at one point along the edge and gradually letting this value change to a negative value -W at the diametrically opposite point on the edge. The value W at one point of the measuring lens is equal to the value W at the opposite point but the strengths are opposite in sign. A second wavefront is applied perpendicular to this first wavefront. The structure of this wavefront is the same as the structure of the first wavefront. This measuring instrument 11 is suitable for measuring coma as a higher order aberration during subjective refraction.

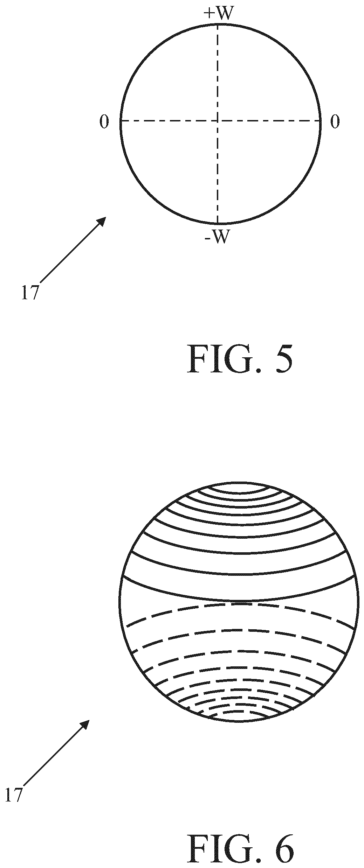

Figure 5 shows a fitting lens of a fitting set that is used in combination with the measuring instrument described above. The fitting lens 17 has a wavefront in one direction with a strength that gradually increases from a value of -W to a value of +W (a wavefront is when the strength increases in one direction). In a direction perpendicular to this wavefront, the fitting lens 17 has a strength of zero (plano).

Figure 6 shows the topography of the fitting lens 17. The wavefront in the fitting lens is optically constructed by giving the strength of the fitting lens a positive value of +W at a location along the edge and gradually letting this value decrease to a negative value of -W at the diametrically opposite location on the edge. The value W at one place of the fitting lens 10 is equal to the value W at the opposite place but the powers are opposite in sign. In the direction perpendicular to this wavefront there is a plano power.

A fitting set of these fitting lenses 17 can be constructed as follows:

| | Horizontal | Vertical at the bottom | Vertical at the top |
|---|---|---|---|
| XW 0,25 | Plano | -0,12 dpt | +0,12 dpt |
| XW 0,50 | Plano | -0,25 dpt | +0,25 dpt |
| XW 0,75 | Plano | -0,37 dpt | +0,37 dpt |
| XW 1,00 | Plano | -0,50 dpt | +0,50 dpt |
| XW 1,25 | Plano | -0,62 dpt | +0,62 dpt |
| XW 1,50 | Plano | -0,75 dpt | +0,75 dpt |
| XW 1,75 | Plano | -0,87 dpt | +0,87 dpt |
| XW 2,00 | Plano | -1,00 dpt | +1,00 dpt |
| XW 2,25 | Plano | -1,12 dpt | +1,12 dpt |
| XW 2,50 | Plano | -1,25 dpt | +1,25 dpt |
| XW 2,75 | Plano | -1,37 dpt | +1,37 dpt |
| XW 3,00 | Plano | -1,50 dpt | +1,50 dpt |
| XW 3,50 | Plano | -1,75 dpt | +1,75 dpt |
| XW 4,00 | Plano | -2,00 dpt | +2,00 dpt |
| XW 4,50 | Plano | -2,25 dpt | +2,25 dpt |
| XW 5,00 | Plano | -2,50 dpt | +2,50 dpt |
| XW 5,50 | Plano | -2,75 dpt | +2,75 dpt |
| XW 6,00 | Plano | -3,00 | +3,00 dpt |

This fitting set of these fitting lenses is suitable for determining the strength of the correction in the case of coma as a higher order aberration during subjective refraction, but not limited by the above-mentioned values.

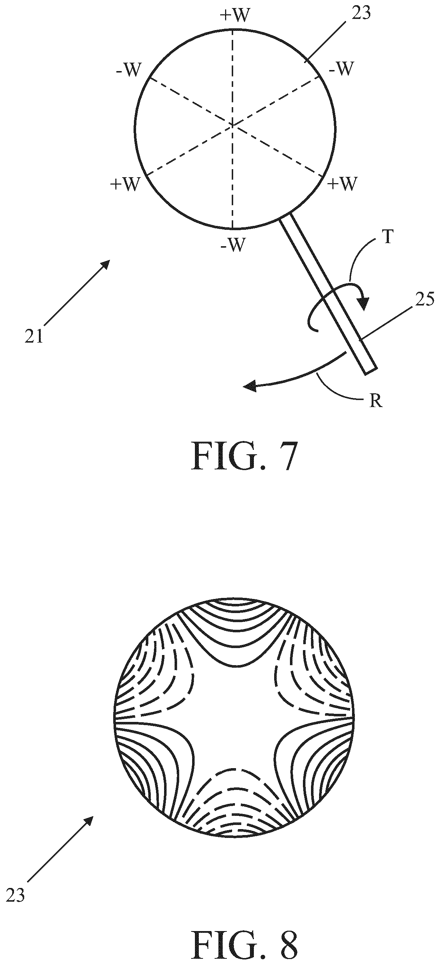

In figure 7 a second embodiment of the measuring instrument 21 according to the invention is shown schematically. The measuring lens 23 of this measuring instrument 21 has in three different directions (with equal angles between these directions), three different wavefronts with strengths that gradually increase from a value -W to a value of +W, whereby the sign of neighbouring directions is different. The handle 25 of this measuring instrument 21 is held up in two different positions each time during the determination of the amount of higher order aberrations to be corrected, which differ by 30 degrees. This can also be used to determine the strength of the wavefront by turning the handle 25 180 degrees around the axis of the handle, arrow T. By turning the position of the handle by 30 degrees in the direction of arrow R, the axis direction of the wavefront is determined by turning the handle again 180 degrees around the axis of the handle.

Figure 8 shows the topography of the measuring lens 23 of the measuring instrument 21. This measuring lens 23 has two different wavefronts in three different directions, the maximum and minimum values of the strengths of which are equal. A wavefront in the measuring lens is optically constructed by giving the measuring lens a positive strength at one point on the edge and a negative strength at the diametrically opposite point on the edge. This construction creates a central plano strength. This measuring lens is suitable for measuring trefoil as a higher order aberration during subjective refraction.

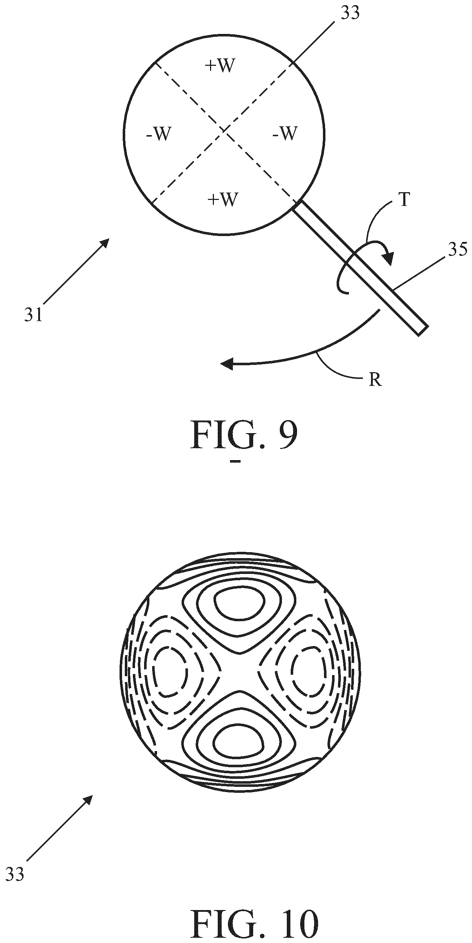

Figure 9 shows a third embodiment of the measuring instrument 31 according to the invention, schematically. The measuring lens 33 of this measuring instrument 31 has two different spherical powers +W and -W in the four quadrants, which are equal in absolute values but differ in sign, where the strength of neighbouring quadrants differs in sign. Here too, the handle 35 is held up in two different positions each time during the determination of the amount of higher order aberrations to be corrected, which differ by 45 degrees. With the handle in the direction of the wavefront, the strength of the wavefront is determined by turning the handle around the axis 180 degrees (arrow T). By turning the position of the handle by 45 degrees (arrow R), the axis direction of the wavefront is determined by turning the handle around the axis 180 degrees (arrow T). Figure 10 shows the topography of the measuring lens 33 of the measuring instrument 31. The measuring lens 33 of this measuring instrument 31 has two different strengths in four quadrants. The strengths are all equal to each other, but positive in one direction and negative in the other direction perpendicular to the first direction. This creates a plano strength between the four quadrants. This measuring lens 33 is suitable for measuring secondary astigmatism as a higher order aberration during subjective refraction.

It should be noted that the shape of the measuring glasses described above according to the invention is bi-convex and bi-concave.

Although the invention has been explained above using the drawings, it should be noted that the invention is by no means limited to the embodiments shown in the drawings. The invention also extends to all embodiments that deviate from the embodiments shown in the drawings within the framework defined by the claims. For example, the maximum value of the positive strength and the absolute value of the minimum value of the negative strength of the varying strength in one direction of the measuring lens can differ from each other instead of being equal to each other. For example, the value of the positive power of the measuring glass at the top may be less than the absolute value of the negative power at the bottom of the measuring glass, such as +0.50 dpt above and -1.50 dpt below.

## Claims

1. Measuring instrument for determining the necessary correction of a lens to reduce the negative effect on vision due to aberrations, comprising a measuring lens having a first strength in a first direction in the plane of the measuring lens and through the centre of the measuring lens and having a second strength in a second direction at an angle to the first direction in the plane of the measuring lens and through the centre of the measuring lens, which measuring instrument further comprises a straight handle connected to the measuring lens and extending in the plane of the measuring lens, the centre line of the handle passing through the centre of the measuring lens and extending along a line dividing an angle between the two said directions into two equal parts, **characterised in that** the measuring lens has a changing strength in the said directions.

2. Measuring instrument according to claim 1, **characterised in that** the measuring lens has a gradually changing strength in the said directions.

3. Measuring instrument according to claim 1 or 2, **characterized in that** the measuring lens has a varying strength from a positive strength to a negative strength in at least one of the said directions.

4. Measuring instrument according to claim 3, **characterized in that** the maximum value of the positive strength and the absolute value of the minimum value of the negative strength are equal to each other.

5. Measuring instrument according to claim 3 or 4, **characterized in that** the measuring lens has a varying strength from a positive strength to a negative strength in at least two directions at an angle to each other.

6. Measuring instrument according to claim 5, **characterized in that** the first and second directions are at right angles to each other.

7. Measuring instrument according to claim 5, **characterized in that** the measuring lens further has a varying strength from a positive strength to a negative strength in the plane of the measuring lens in a third direction at an angle to the first and second directions, whereby the variation of the strength in two adjacent directions is opposite to each other.

8. Measuring instrument according to claim 1 or 2, **characterized in that** quadrants of the measuring lens have two different spherical powers that are equal in absolute values but differ in sign, whereby the powers of adjacent quadrants differ in sign.

9. Assembly of a measuring instrument according to claim 6 and a set of fitting lenses, each fitting lens having a power varying from a positive power to a negative power in a first direction in the plane of the fitting lens and through the centre of the fitting lens, the maximum value of the positive power and the absolute value of the minimum value of the negative power being equal to each other, and which fitting lens has no power in a second direction in the plane of the fitting lens and through the centre of the fitting lens at an angle to the first direction.
